# EUROPEAN PATENT APPLICATION

(11) **EP 1 598 669 A1**
(43) Date of publication of application: **23.11.2005**
(21) Application number: 04011768.1
(22) Date of filing: 18.05.2004
(51) Int. Cl.: G01N 33/543, G01N 27/00

(54) **Bio-vehicle, biosensor and biotransducer system**

(71) Applicant: Exon Science Inc., Guei Shan Hsiang&Taoyuan,Taiwan 333 (TW)
(72) Inventor: Hsieh, Shih-Yang, N. Rd., Shrlin chiu, Taipei, Taiwan 111 (TW); Lin, Ming-Tse, Taipei, Taiwan 241 (TW); Su, Wen-Wei, Guei Shan Hsiang, Taoyuan, Taiwan (TW); Chen, Kuei-Hung, Guei Shan Hsiang. Taoyuan, Taiwan (TW); Liu, Po-Chang, Rd., Daan Chiu, Taipei, Taiwan 106 (TW)
(74) Representative: Winkler, Andreas Fritz Ernst

(57) **Abstract**

A bio-electronic system for detecting a broad range of chemicals is achieved by the incorporation of ligand-binding affinities of lipocalins with a mass-sensing electronic device. A biotransducer system combines the unique ligand-binding affinities of lipocalins with the high sensitivity of piezoelectric-based devices. As "artificial-nose", this system is more reliable, cost-effective, and flexible for a number of applications in sensory evaluation, including air quality monitoring, quality control of cosmetic and fermentation products, medical diagnosis via breath analysis *et al*. These proteinaceous elements are likely to be extended to several potential applications, e.g., for the purification and delivery of specific ligands.

## Description

The present invention relates to a bio-vehicle, and more particularly to a bio-vehicle which is proteinaceous and capable of capturing and preserving chemicals of low molecular weight, e.g. odorants, drugs, and pigments. The present invention also relates to a biosensor, and more particular to a biosensor comprising a proteinaceous bio-vehicle and capable of detecting the binding of chemicals to the bio-vehicle. The present invention further relates to a biotransducer system, and more particularly a biotransducer system comprising a proteinaceous bio-vehicle and generating an electronic signal while detecting the binding of chemicals to the bio-vehicle. The present invention also relates to the uses of the above devices.

To date, commercially available instruments in the artificial-olfaction field are rather sophisticated and most of them are expensive. Conventional array-based chemical sensing has followed a one-sensor-one-analyte approach that suffers on several disadvantages, e.g., highly selective binding materials for wanted ligands are rare and difficult to find. In fact, the detecting specificity of current systems is limited to a small number of ligands. Moreover, the outputs need to be processed by a PC-like hardware and often become extremely complicated when multiple analytes are applied. Several 'artificial-nose' systems have been demonstrated mainly based on chemical-sensing techniques; however, practical applications in current platforms are prevented by the high cost and limited information given. An alternative paradigm for sensing is to mimic the biological system, which has been shown in the booming of biosensors (sensors incorporating a biological or biologically derived sensing elements). For designing the second-generation biosensor to satisfy more demands, ranged form home testing to the on-line monitoring in manufactories, the pocket-sized glucose biosensors already in market are considerable standards. Accordingly, portable instruments available at present (or in the expecting future) may allow users to design their own biosensors by replacing the chips of sensing elements. Such pioneer devices can be exampled by the "HAZMATCAD" manufactured by the Microsensor Systems Inc. (USA), the "Cyranose 320" provided by the Cyrano Sciences Inc. (Canada), and the "Chem Lab on a Chip" announced by the Sandia National Laboratories (USA). Despite of that, extensive applications of artificial-nose are still constrained by the variety of sensing elements that can be fabricated in scale and sold in cheap.

Odor perception in biological system first occurs during odorant molecules binding onto specific protein receptors within specialized organs or tissues. In one approach, biological materials have to be utilized as parts of electronic nose devices based on different sensing technologies; therefore, the selections of biological materials are quite diverse. Preferable biomaterials can be purified olfactory receptors (Wu, Biosens. Bioelectron. 14:9 (1999)), antibodies (Wong et al., Biosens. Bioelectron. 17:9 (2002)), fish scales (Lundstrom and Svensson, Biosens. Bioelectron. 13:689 (1998)), insect antennae (Schroth et al., Electrochimica Acta 44: 3821 (1999)), and synthetic peptides (Wu and Lo, J. Biotech. 80:63 (2000)). Unfortunately, all these systems seem have difficulties for production in the industrial manner, considering the cost and viability. Consequently, there is a need to seek the ideal biomaterials for a broad range of sensing applications.

An objective of the present invention is to provide a bio-vehicle that provides alternative options for capturing chemicals such odorants, drugs, and pigments.

Another objective of the present invention is to provide a biosensor that can be fabricated in scale and sold in cheap.

A further objective of the present invention is to provide a biotransducer system which can be applied to a broad range of sensing applications.

A first aspect of the present invention relates to a bio-vehicle for binding a specified ligand. The bio-vehicle comprises a solid support; and a proteinaceous substrate received by the solid support, and capable of binding the specified ligand. The proteinaceous substrate includes a protein selected from a group consisting of lipocalin proteins and recombinant functional homologues thereof.

The solid support, for example, can be a disposable biochip or a lasting container.

The bio-vehicle according to the present invention is especially suitable for binding the ligand having a molecular weight of less than 1000 daltons. The ligand, for example, can be an odorant, a drug or a pigment. Examples include 2-phenylethyl alcohol, 2-phenylethyl acetate, phenylacetate, propylbutyrate, hexylbutyrate, benzylbutyrate, propyltiglate, hexyltiglate, and benzyltiglate.

One of the examples of the proteinaceous substrate is major urinary protein (MUP).

A second aspect of the present invention relates to a biosensor for detecting the binding of a specified ligand. The biosensor comprises a piezoelectric base producing electrical charges in response to a mechanical stress; and a proteinaceous substrate immobilized on the piezoelectric base, and providing the mechanical stress to the piezoelectric base upon binding the specified ligand.

A third aspect of the present invention relates to a biotransducer system. The biotransducer system comprises a proteinaceous substrate for binding a specified ligand; and an electronic detecting device in connection with the proteinaceous substrate for detecting the binding of the specified ligand to the proteinaceous substrate, and generating an electronic signal to indicate the presence of the specified ligand.

The proteinaceous substrate preferably includes a protein selected from a group consisting of lipocalin proteins and recombinant functional homologues thereof. The electronic detecting device preferably includes a piezoelectric-based device and a recorder.

The piezoelectric-based device, for example, can be a quartz crystal microbalance (QCM) or surface acoustic wave (SAW) device.

A further aspect of the present invention relates to a method for determining the binding of a specified ligand to a proteinaceous substrate. The method comprises steps of providing a piezoelectric base; immobilizing a proteinaceous substrate onto the piezoelectric base; exposing the proteinaceous substrate and the piezoelectric base to an environment where the specified ligand exists; and detecting a resonant frequency variation of the piezoelectric base to determine whether the specified ligand is bound to the proteinaceous substrate.

A fifth aspect of the present invention relates to the use of the bio-vehicle for absorption, preservation, purification or delivery of the specified ligand.

A sixth aspect of the present invention relates to the use of the biotransducer system in an application of sensory evaluation, air quality monitoring, quality control of cosmetic products, quality control of fermentation products or medical diagnosis via breath analysis.

The present invention may best be understood through the following description with reference to the accompanying drawings, in which:
Fig. 1 is a schematic block diagram showing the main portions of a transducer system;
Fig. 2 is a frequency vs. time diagram illustrating the electronic signals outputted by the biotransducer system according to a preferred embodiment of the present invention, wherein air is introduced into the system for blank test;
Fig. 3 is a frequency vs. time diagram illustrating the electronic signals outputted by the same biotransducer system as that of Fig. 1, wherein a high concentration of ligand sample is introduced into the system for binding test;
Fig. 4 is a frequency vs. time diagram illustrating the electronic signals outputted by the same biotransducer system as that of Fig. 1, wherein a low concentration of ligand sample is introduced into the system for binding test;
Fig. 5 is a frequency vs. time diagram illustrating the electronic signals outputted by the same biotransducer system as that of Fig. 1, wherein a low and then a high concentration of ligand samples are introduced into the system for binding test;
Fig. 6 is a frequency vs. time diagram of an example of the present biotransducer system, which demonstrates the binding of an evaporated odorant produced by a yeast culture at the early stage of fermentation and
Fig. 7 is a frequency vs. time diagram of an example of the present biotransducer system, which demonstrates the binding of the evaporated odorant produced by the same yeast culture as in Fig. 5 at the middle stage of fermentation .

The present invention will now be described more specifically with reference to the following embodiments. It is to be noted that the following descriptions of preferred embodiments of this invention are presented herein for purpose of illustration and description only; it is not intended to be exhaustive or to be limited to the precise form disclosed.

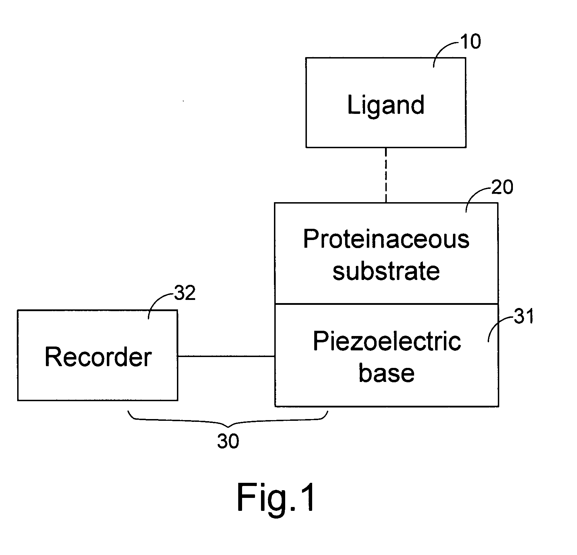

Referring to Fig. 1, a biotransducer system comprises one or more groups of proteinaceous elements capable of binding a broad range of chemicals of ligands 10 as a proteinaceous substrate 20, and an electronic detecting device 30 generating electronic signals varying with features of ligands. The electronic detecting device 30 includes a piezoelectric base 31 producing electric charges in response to a mechanical stress and a recorder 32 electrically connected to the piezoelectric base 31. This system is aimed at but not limited to a simple and portable device that can be used in routine works for sensing evaluation.

The proteinaceous substrate 20 is immobilized onto the piezoelectric base 31. When the ligand(s) is bound to the proteinaceous substrate 20, a mechanical stress is applied onto the piezoelectric base 31 so that electric charges are generated and the recorder 32 records the electrionic signals accordingly.

For the application of sensory evaluation, air quality monitoring, quality control of cosmetic products, quality control of fermentation products or medical diagnosis via breath analysis, the biotransducer system is preferably capable of binding a ligand chemical(s) 30 of odorants, drugs, and pigments.

The protein or proteins included in the proteinaceous substrate 20 is prferably selected from a lipocalin protein family and recombinant functional homologues thereof. A profound review of lipocalin can be seen in Biochimica et Biophysica Acta (BBA)- Protein Structure and Molecular Enzymology 1482 (2000)).

Lipocalins are a prolific group of proteins (more than 140) found in eubacteria and a variety of eukaryotic cells. Their major biological functions are pheromonal carriers, transporters for vitamin A, olfactory and gustatory proteins, colouration pigments, synthases of prostaglandin D, accessory enzymes for photosynthesis, modulators of immunity, stress proteins, and regulators of cell differentiations. Despite of low levels of overall sequence similarity (usually below 20%), members of the lipocalin family share a common signature of an eight-stranded antiparallel -barrel with a repeated +1 topology enclosing an internal ligand-binding pocket (for a concise introduction: www.jenner.ac.uk/lipocalin.htm).

Most of lipocalins are abundant and can be isolated from the natural sources, e.g., one lipocalin, beta-lactoglobulin (BLG), is the major protein contain of cow's milk. Recombinant forms of lipocalins are available and well known in the art, e.g., retinal-binding protein (Wang et al., Gene 133(2):291 (1993)), tear lipocalin (Holzfeind et al., FEBS Lett. 395(2-3):95 (1996)), bovine beta-lactoglobulin (Cho et al., Protein Engineering 7(2):263 (1994)), major urinary protein (Ferrari et al., FEBS Lett. 401(1):73 (1997)), nitric oxide transport protein (Andersen et al., Structure 6(10):1315 (1998)), and odorant-binding protein (Lobel et al., European J. Biochem. 254(2):318 (1998)).

One of the examples of the lipocalins is the major urinary protein (MUP). MUP is the major protein component of mouse urine (about 5-10 mg per day for male) and acts as pheromone transporter (for review see Cavaggioni and Mucignat-Caretta, BBA 1482(2-1):218 (2000)). Recombinant MUP can be prepared by a standard procedure well known to those skilled in the art. MUP performs especially well for carrying compounds of lower molecular weight, e.g. less than 1000 daltons and very volatile.

An embodiment of the piezoelectric base 31 is a quartz crystal microbalance (QCM).

The preferred electronic nose technology of the present invention is piezoelectric-based. Measurements are performed by detecting the binding of analytes (ligands) onto the sensing elements immobilized on piezoelectric materials, e.g., quartz crystal. The adsorption of ligand induces a shift in the oscillation frequency proportional to the mass of the encountered ligand. The operating performance of the present invention is described as bellow:
i) Surface activation of QCM chip
   Gold electrodes of quartz crystal microbalance (QCM) surface are cleaned by immersing in 1.2 N NaOH and then in 1.2 N HCl. After rinsing and drying, the QCM electrodes are soaked in 1 mM 2-aminoethanethiol/DMSO solution for 12 hours and washed by double distilled water for 10 min. Then the QCM electrodes are dried in oven and dripped with 4 µl of 2.5% glutaldehyde for 1 hour. Two µl of 1% polyethylenimine/methanol (PEI/MeOH) solution are added onto activated surface of QCM in a closed chamber at room temperature for 12 hours. Finally, the QCM chips are washed two times and dried in oven.
ii) Immobilization of MUP on activated QCM chips
   Activated QCM chips are dipped in 2.5% glutaldehyde solution for 1 hour, and then MUP solution (20µg/ml in 10mM phosphate buffer, pH 7.4) are added and kept for 1 hour. Blocking the activated surface of QCM chips is performed by 100 mM glycine.
iii) Determining the binding of ligand
   A sample is respectively applied in a syringe and kept in an oven at 50°C. An AT-cut QCM device with basic resonant frequency of 10MHz is employed to determine the adsorption of odorant vapor. The evaporated odorant sample is pumped into the detection chamber of QCM and the shift of resonant frequency is recorded.

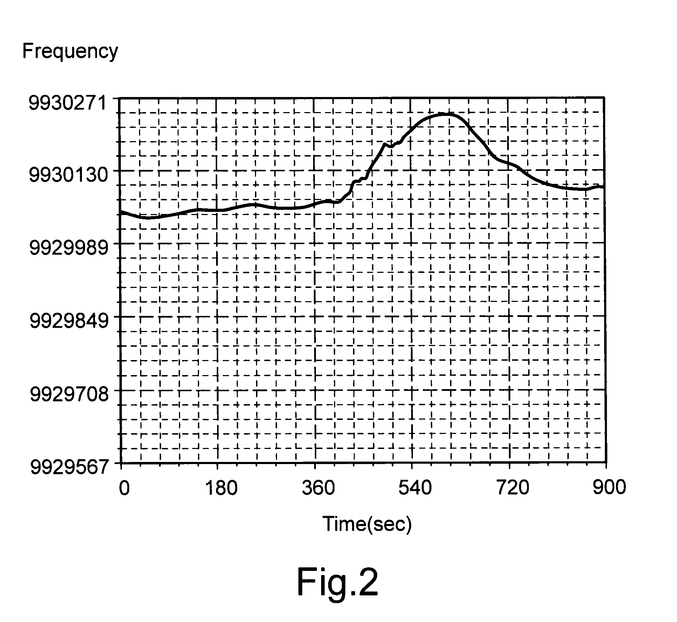

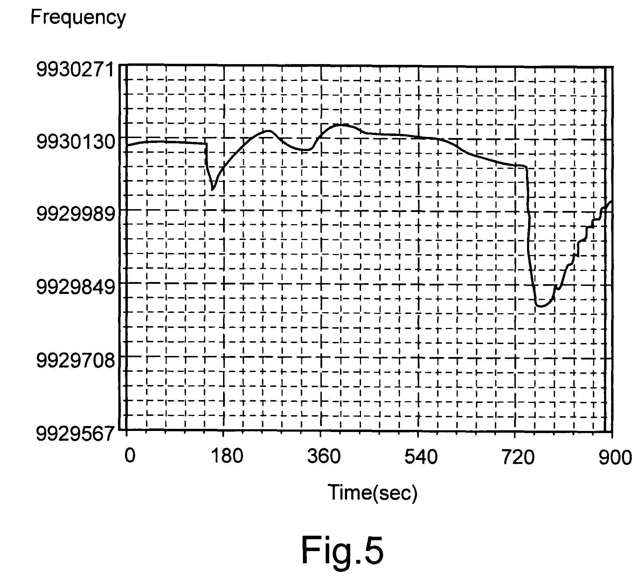

Figs. 2~5 illustrates the frequency vs. time diagrams of various samples illustrating the electronic signals outputted by the same biotransducer system.

The sample for the ligand-binding test in Fig. 2 is air. Since a flow rate of air results in a certain level of buoyancy, the resonant frequency of QCM increases.

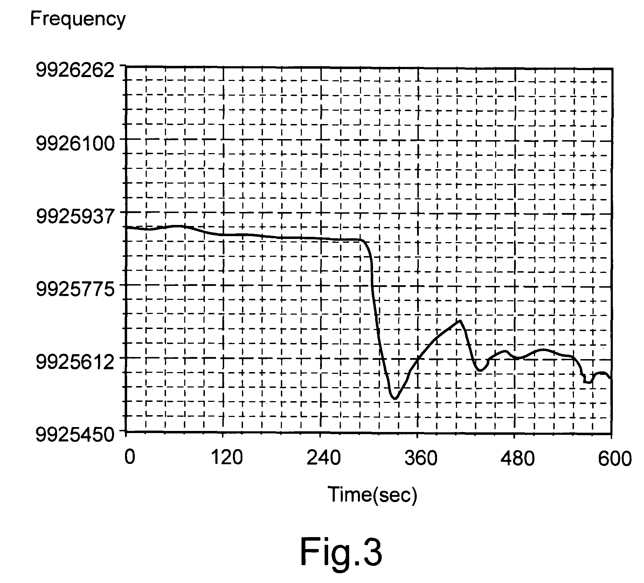

The samples for the ligand-binding test in Figs. 2 and 3 are 40 µl and 4 µl phenylacetate, respectively. As shown, the adsorption of ligand induces a shift in the oscillation frequency proportional to the mass of the encountered ligand. It is also shown that a diluted sample can also be detected by the present system.

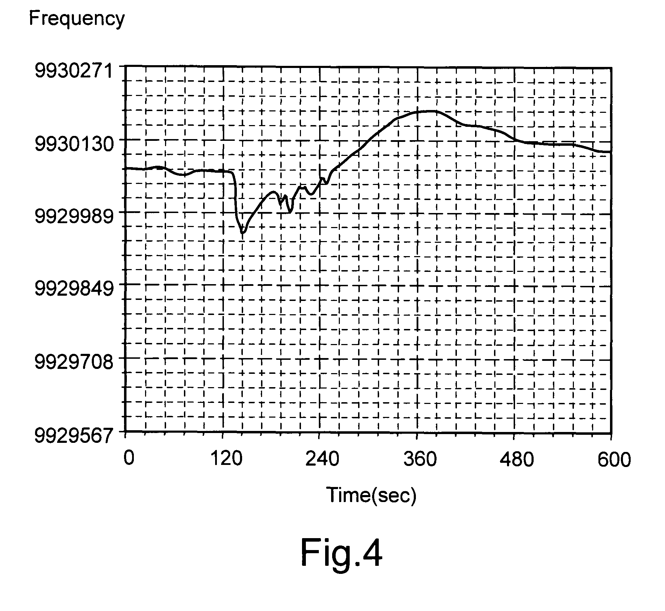

The samples for the ligand-binding test in Fig. 4 are 4 µl phenylacetate, air and 40 µl phenylacetate in sequence. It is shown that the samples can be adsorbed and desorbed as well. Therefore, the present system can be reused and has potential in quantitative analysis and on-line determination.

For the biochemical vapor testing, gaseous samples corresponding to 10 cm³ of the yeast culture exposure are analyzed by the present invention. *Pichia fermentans* L-5 is cultured in 4 L fermentor with 1.6 L working volume aerated in 0.6 vvm (volume air/volume tank per minutes) at 400 rpm. The initial medium contains phenylalanine (2000 ppm), glucose (100 g/L), and 50 mM KH₂PO₄. The expected odorants in such culture exposure includes 2-phenylethyl alcohol and 2-phenylethyl acetate et al.

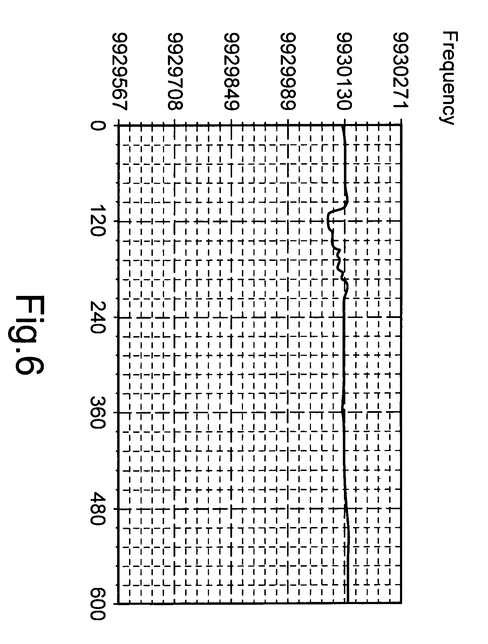

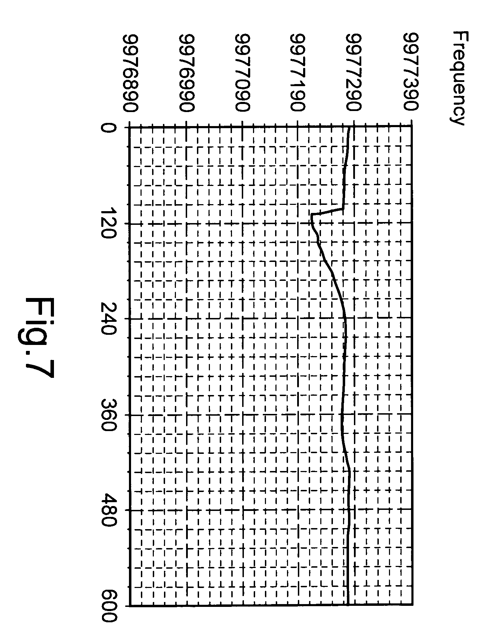

Figs. 6 and 7 shows the binding of the odorants onto the proteinaceous substrate at the early stage and middle stage, respectively. The potential in quantitative analysis and on-line determination is prominent.

A variety of ligands are surveyed according to the above procedures. Table I lists the changes of signal in frequency upon the bindings of different ligands, including phenylacetate, propylbutyrate, hexylbutyrate, benzylbutyrate, propyltiglate, hexyltiglate, and benzyltiglate.

**Table I**

| Ligand | phenylacetate | propylbutyrate | hexylbutyrate | benzylbutyrate |
|---|---|---|---|---|
| Change of | | | | |
| Signal (in | 46 | 26 | 23 | 43 |
| frequency *f*) | | | | |

| Ligand | propyltiglate | hexyltiglate | benzyltiglate | |
|---|---|---|---|---|
| Change of | | | | |
| Signal (in | 29 | 55 | 43 | |
| frequency *f*) | | | | |

In addition to QCM, the electronic detecting device used in the present system can also be a field effect transistor (FET) device or surface acoustic wave (SAW) device. The principle and operation of the devices are well known to those skilled in the art, and will not redundantly described herein.

In addition to the application in a biosensor or biotransducer system, the proteinaceous substrate can be used as a bio-vehicle for absorption, preservation, purification or delivery of specified ligands. The proteinaceous substrate is immobilized onto or received in a solid support (not shown) which can be a disposable biochip or a lasting container.

While the invention has been described in terms of what are presently considered to be the most practical and preferred embodiments, it is to be understood that the invention need not be limited to the disclosed embodiment. On the contrary, it is intended to cover various modifications and similar arrangements included within the spirit and scope of the appended claims which are to be accorded with the broadest interpretation so as to encompass all such modifications and similar structures.

The features disclosed in the foregoing description, in the claims and/or in the accompanying drawings may, both separately and in any combination thereof, be material for realizing the invention in diverse forms thereof.

## Claims

1. A bio-vehicle for binding a specified ligand, **characterized by** comprising:
a solid support; and
a proteinaceous substrate received by the solid support, and capable of binding the specified ligand;
wherein the proteinaceous substrate includes a protein selected from a group consisting of lipocalin proteins and recombinant functional homologues thereof.

2. The bio-vehicle according to claim 1, **characterized in that** the solid support is a disposable biochip or a lasting container.

3. The bio-vehicle according to claim 1, **characterized in that** the specified ligand has a molecular weight of less than 1000 daltons.

4. The bio-vehicle according to claim 1, **characterized in that** the specified ligand is one selected from a group consisting of odorants, drugs, and pigments.

5. The bio-vehicle according to claim 1, **characterized in that** the specified ligand is one selected from a group consisting of 2-phenylethyl alcohol, 2-phenylethyl acetate, phenylacetate, propylbutyrate, hexylbutyrate, benzylbutyrate, propyltiglate, hexyltiglate, and benzyltiglate.

6. The bio-vehicle according to claim 1, **characterized in that** the proteinaceous substrate includes major urinary protein (MUP).

7. The bio-vehicle according to claim 1, **characterized by** being used for absorption, preservation, purification or delivery of the specified ligand.

8. A biosensor for detecting the binding of a specified ligand,
**characterized by** comprising:
a piezoelectric base producing electrical charges in response to a mechanical stress; and
a proteinaceous substrate immobilized on the piezoelectric base, and providing the mechanical stress to the piezoelectric base upon binding the specified ligand.

9. The biosensor according to claim 8, **characterized in that** the piezoelectric base is a quartz crystal microbalance (QCM) or a surface acoustic wave (SAW) base.

10. The biosensor according to claim 8, **characterized in that** the proteinaceous substrate includes a protein selected from a group consisting of lipocalin proteins and recombinant functional homologues thereof.

11. The biosensor according to claim 10, **characterized in that** the proteinaceous substrate includes major urinary protein (MUP).

12. The biosensor according to claim 11, **characterized in that** the specified ligand is one selected from a group consisting of 2-phenylethyl alcohol, 2-phenylethyl acetate, phenylacetate, propylbutyrate, hexylbutyrate, benzylbutyrate, propyltiglate, hexyltiglate, and benzyltiglate.

13. The biosensor according to claim 8, **characterized in that** the specified ligand is one selected from a group consisting of odorants, drugs, and pigments.

14. A biotransducer system for detecting the presence of a specified ligand, **characterized by** comprising:
a proteinaceous substrate for binding a specified ligand; and
an electronic detecting device in connection with the proteinaceous substrate for detecting the binding of the specified ligand to the proteinaceous substrate, and generating an electronic signal to indicate the presence of the specified ligand.

15. The biotransducer system according claim 14, **characterized in that** the specified ligand is one selected from a group consisting of odorants, drugs, and pigments.

16. The biotransducer system according claim 14, **characterized in that** the specified ligand is one selected from a group consisting of 2-phenylethyl alcohol, 2-phenylethyl acetate, phenylacetate, propylbutyrate, hexylbutyrate, benzylbutyrate, propyltiglate, hexyltiglate, and benzyltiglate.

17. The biotransducer system according to claim 16, **characterized in that** the proteinaceous substrate includes major urinary protein (MUP).

18. The biotransducer system according to claim 14, **characterized in that** the proteinaceous substrate includes a protein selected from a group consisting of lipocalin proteins and recombinant functional homologues thereof.

19. The biotransducer system according to claim 14, **characterized in that** the electronic detecting device includes a piezoelectric-based device and a recorder.

20. The biotransducer system according to claim 19, **characterized in that** the piezoelectric-based device is one selected from a group consisting of a quartz crystal microbalance (QCM) and surface acoustic wave (SAW) device.

21. The biotransducer system according to claim 14, **characterized by** being used in an application of sensory evaluation, air quality monitoring, quality control of cosmetic products, quality control of fermentation products or medical diagnosis via breath analysis.

22. A method for determining the binding of a specified ligand to a proteinaceous substrate, **characterized by** comprising steps of:
providing a piezoelectric base;
immobilizing a proteinaceous substrate onto the piezoelectric base;
exposing the proteinaceous substrate and the piezoelectric base to an environment where the specified ligand exists; and
detecting a resonant frequency variation of the piezoelectric base to determine whether the specified ligand is bound to the proteinaceous substrate.
